# EUROPÄISCHE PATENTSCHRIFT

(11) **EP 3 682 870 B1**
(45) Veröffentlichungstag und Bekanntmachung des Hinweises auf die Patenterteilung: **30.08.2023**
(21) Anmeldenummer: 19151878.6
(22) Anmeldetag: 15.01.2019
(51) Int. Cl.: A61K 9/20, A61K 31/00

(54) **VERFAHREN ZUR HERSTELLUNG VON LEICHT EINZUNEHMENDEN TABLETTEN MIT TROCKENEXTRAKT AUS GINKGO BILOBA BLÄTTERN**
METHOD FOR THE PREPARATION OF EASILY CONSUMABLE TABLETS CONTAINING GINKGO BILOBA LEAF EXTRACT
PROCÉDÉ DE FABRICATION DE COMPRIMES FACILES À PRENDRE COMPRENANT UN EXTRAIT SEC DES FEUILLES DE GINKGO BILOBA

(43) Veröffentlichungstag der Anmeldung: 22.07.2020
(73) Patentinhaber: Dr. Willmar Schwabe GmbH & Co. KG, 76227 Karlsruhe (DE)
(72) Erfinder: HERRMANN, Joachim, 76227 Karlsruhe (DE); ROTHE, Andreas, 76227 Karlsruhe (DE)
(74) Vertreter: Adam, Holger

(56) Entgegenhaltungen:
- EP-A1- 2 072 054
- ANONYMOUS: "Tebonin intens 120 mg", GEBRAUCHSINFORMATION: INFORMATION FÜR DEN ANWENDER, 1. Februar 2014 (2014-02-01), Seiten 1-4, XP055528590,

## Beschreibung

Die vorliegende Erfindung betrifft ein Verfahren zur Herstellung einer schnell zerfallenden Tablette mit einer Zerfallszeit von höchstens 15 Minuten zur peroralen Verabreichung eines Trockenextraktes aus den Blättern von Ginkgo biloba und mit einem Gesamtgewicht der Tablette zwischen 150 mg und 300 mg pro 100 mg des enthaltenen Ginkgoextrakts. Gegenstand der Erfindung sind ferner schnell zerfallende Tabletten mit Trockenextrakt aus den Blättern von Ginkgo biloba, die aufgrund ihrer kleineren Abmessungen leichter einzunehmen sind als die bisher verwendeten Tabletten. In einer bevorzugten Ausführungsform enthalten die Tabletten keine Laktose und sind daher zudem gut verträglich.

Extrakte aus den Blättern von Ginkgo biloba werden seit Jahrzehnten als Arzneimittel verwendet. Derzeit werden sie zur Behandlung verschiedener Arten von Demenz und deren Symptomen sowie von cerebralen und peripheren Durchblutungsstörungen, Tinnitus und Schwindel verwendet. Inhaltsstoffe mit denen die Wirksamkeit verknüpft ist, sind Terpenlaktone (Ginkgolide A, B, C und Bilobalid) sowie Glycoside von Flavonen (Quercetin, Kämpferol und Isorhamnetin). Gemäß Ph. Eur. enthält arzneilich verwendeter Ginkgotrockenextrakt 22,0 bis 27,0 % Flavonoide berechnet als Flavonglycoside, 2,6 bis 3,2 % Bilobalid, 2,8 bis 3,4 % Ginkgolide A, B und C und höchstens 5 ppm Ginkgolsäuren. Gemäß dem Europäischen Arzneibuch haben Trockenextrakte im Allgemeinen einen Trocknungsverlust von höchstens 5 Gew.-% entsprechend einem Trockenrückstand von mindestens 95 Gew.-%". Der in Tebonin^{®} enthaltene Spezialextrakt EGb761^{®} entspricht ebenfalls dieser Spezifikation. Diese dementiellen Erkrankungen betreffen weit überwiegend Menschen im höheren Lebensalter, die oftmals eine Vielzahl verschiedener Medikamente einnehmen müssen und unter Schluckbeschwerden leiden, bedingt durch die mit zunehmendem Alter abnehmende Produktion von Speichelflüssigkeit und häufig das Vorliegen bestimmter zusätzlicher neurologischer Erkrankungen, wie z.B. Morbus Parkinson. Diese Probleme bei der Einnahme von Arzneimitteln führen oftmals zu einer verringerten Therapietreue durch nicht eingenommene Arzneimittel und in der Folge dem Ausbleiben des medikamentösen Behandlungserfolges.

Nach der Definition des Europäischen Arzneibuches 9. Ausgabe 2017 (Ph.Eur.) sind Tabletten feste Arzneizubereitungen, die eine Einzeldosis eines oder mehrerer Wirkstoffe enthalten. Tabletten (ohne Überzug) müssen der Prüfung 2.9.1. "Zerfallszeit" des Europäischen Arzneibuches entsprechen und unter den Testbedingungen innerhalb von 15 Minuten zerfallen. Zur Verbesserung der Stabilität, der Sicherstellung der Unterscheidbarkeit und zur Verbesserung der Schluckbarkeit werden Tabletten meist mit einem eingefärbten Film überzogen. Ist der Film ein sehr dünner Polymerüberzug, werden die Tabletten als Filmtabletten bezeichnet. Filmtabletten müssen gemäß Ph.Eur. innerhalb von 30 Minuten zerfallen.

Den Vorteilen von Tabletten wie z.B. die genaue Dosierung stehen auch Nachteile gegenüber, z.B. die Notwendigkeit, dass sie als fester Fremdkörper unzerkaut geschluckt werden und innerhalb von 15 Minuten zerfallen müssen, um ihre Wirkung entfalten zu können. Eine kleine Tablettengröße und eine kurze Zerfallszeit sind somit wichtige Eigenschaften. Beides zu erreichen kann problematisch sein, da Tabletten durch Verpressen eines definierten Volumens von Teilchen hergestellt werden und kleine, hart gepresste Tabletten mit einem hohen prozentualen Massenanteil an Arzneiwirkstoff physikalisch bedingt zwangsläufig eine längere Zerfallszeit aufweisen als größere Tabletten, die einen höheren prozentualen Massenanteil an Hilfsstoffen wie Zerfallsbeschleuniger und Füllmittel und einen kleineren Anteil an Arzneiwirkstoff enthalten. Eine kleine Tablettengröße im Verhältnis zur enthaltenen Wirkstoffmenge ist umso vorteilhafter, je größer die Menge des enthaltenen Wirkstoffs ist. Besondere Relevanz besteht daher im Fall der 240 mg-Tablette, der auf dem Markt befindlichen mit dem höchsten Wirkstoffgehalt. Sollte zukünftig die Bereitstellung von Tabletten mit mehr als 240 mg Ginkgoextrakt erforderlich werden, gewinnt die Verfügbarkeit kompakter Tabletten noch größere Bedeutung.

In der Roten Liste (online Ausgabe 2017) findet man für den Wirkstoff Ginkgo 35 verschiedene Produkte. Davon sind 28 blattextrakthaltige Monoprodukte und die übrigen sieben homöopathische Arzneimittel, die nach homöopathischen Regeln hergestellte Tinkturen oder Verdünnungen enthalten. Bei den Darreichungsformen der Ginkgoblattextrakt-Produkte überwiegen die Filmtabletten mit 25 Produkten, daneben gibt es noch drei Produkte als Flüssigkeiten (Tropfen). Die Filmtabletten enthalten 30, 40, 50, 60, 80, 120 oder 240 mg Trockenextrakt aus den Blättern von Ginkgo biloba. Alle in diesem Deutschen Arzneimittelverzeichnis aufgeführten, bisher bekannten Produkte in der festen Darreichungsform "Filmtablette" enthalten den Bestandteil Laktose oder Laktose-Monohydrat als Teil der Zusammensetzung, wie aus den Produktunterlagen zu entnehmen ist.

In der nachstehenden Tabelle 1 sind die in Deutschland im Markt vertriebenen Produkte mit 240 mg Trockenextrakt aus Ginkgo-Blättern, ihre Abmessungen und ihr Gewicht aufgelistet. Gemäß der jeweiligen Packungsbeilage enthalten alle Produkte Laktose. Bei allen genannten Tabletten handelt es sich um schnell zerfallende Filmtabletten mit einer Zerfallszeit von höchstens 30 Minuten.

**Tabelle 1: Abmessungen und Gewichte der derzeit in Deutschland vertriebenen Produkte**

| Produktbezeichnung | Abmessungen Länge/Breite/ Dicke [mm] | Masse Tablette **ohne Überzug*** [mg] | Verhältnis der Masse der Tablette zur Masse des Wirkstoffs | Masse der **überzogenen** Tablette [mg] |
|---|---|---|---|---|
| Binko 240 | 19,2/8,1/6,1 | Mindestens 781* | 3,25 | 839 |
| Doppelherz^{®} Ginkgo 240 | 19,3/8,2/6,3 | Mindestens 780* | 3,25 | 837 |
| Gingobeta 240 | 19,2/8,1/6,0 | Mindestens 796* | 3,32 | 846 |
| Ginkgo AL 240 | 19,2/8,1/6,1 | Mindestens 768* | 3,20 | 824 |
| Gingium^{®} extra 240 | 20,2/9,2/5,8 | Mindestens 780* | 3,25 | 814 |
| Ginkgovital^{®} Heumann 240 | 19,3/8,2/6,1 | Mindestens 793* | 3,30 | 844 |
| Ginkgo maren^{®} 240 | 19,2/9,0/5,6 | Mindestens 811* | 3,38 | 855 |
| Ginkgo STADA^{®} 240 | 19,2/8,1/6,1 | Mindestens 784* | 3,27 | 838 |
| Ginkobil^{®} ratiopharm 240 | 20,2/9,2/5,8 | Mindestens 780* | 3,25 | 814 |
| Tebonin^{®} konzent 240 | 19,2/8,2/6,0 | 780 | 3,25 | 814 |

| | | | | |
|---|---|---|---|---|
| *Gemessen an den vom Überzug durch Abschälen befreiten Tablettenkernen | | | | |

Der Massenanteil an Wirkstoff an der Gesamtmasse der nicht-überzogenen Tablette beträgt bei den in Deutschland auf dem Markt befindlichen Tabletten zwischen 31,3 und 29,6 % bzw. das Verhältnis von Tablettenmasse zu enthaltener Wirkstoffmasse (TM/WM) 3,20 - 3,38 entsprechend einer Tablettenmasse von 320 mg bis 338 mg pro 100 mg des enthaltenen Ginkgoextrakts.

Aus der Gebrauchsinformation "Tebonin intens 120 mg" (XP055528590) sind ebenfalls schnell zerfallende Tabletten zur peroralen Verabreichung eines Trockenextrakts aus Ginkgo biloba bekannt.

Die EP2072054A1 beschreibt eine Tablette mit 240 mg Trockenextrakt aus Ginkgo biloba Blättern und einer Masse des Tablettenkerns von 800 mg (d.h. mit einem TM/WM = 3,33 entsprechend einer Tablettenmasse von 333 mg pro 100 mg des enthaltenen Ginkgoextrakts), davon 160 mg Laktose-Monohydrat (siehe Vergleichsbeispiel 1 und EP2072054A1, erfindungsgemäßes Beispiel 3). Bei der beschriebenen Tablette handelt es sich um eine Tablette mit schneller Wirkstofffreisetzung (EP2072054A1, erfindungsgemäßes Beispiel 3 "Conventionalrelease dosage form" und Anspruch 15).

Laktose ist ein in Milch vorkommendes Disaccharid bestehend aus Galaktose und Glukose. In wasserfreier Form ist Laktose hygroskopisch; aus der wässrigen Lösung kristallisiert die stabilere α-Form als Monohydrat aus. Laktose ist die am häufigsten verwendete Grundsubstanz für Tabletten ("Die Tablette"; W.A. Ritschel, A. Bauer-Brandl; ECV Verlag Aulendorf, 2002, S. 74). Bei einer Laktoseintoleranz wird der mit der Nahrung aufgenommene Milchzucker als Folge fehlender oder verminderter Produktion des Verdauungsenzyms Laktase nicht oder unvollständig verdaut. Die im Dünndarm nicht verdaute Laktose gelangt bei laktoseintoleranten Personen in den Dickdarm und wird dort von der Darmflora als Nährstoff vergoren. In der Folge kommt es vor allem zu Blähungen, Bauchdrücken, Bauchkrämpfen, Übelkeit, Erbrechen und häufig auch zu spontanen Durchfällen.

Die WO2012/146592A1 (erteilt als EP2701688B1) beschreibt eine Tablette mit 240 mg Trockenextrakt aus Ginkgo biloba Blättern mit kontrollierter Freisetzung und deren Herstellung (siehe Vergleichsbeispiel 2). Diese Tablette ist laktosefrei, wiegt 420 mg, enthält sehr wenig Hilfsstoffe, hat einen Wirkstoffanteil von 57 % m/m (TM/WM = 1,75 entsprechend einer Tablettenmasse von 175 mg pro 100 mg des enthaltenen Ginkgoextrakts) und ist damit sehr kompakt. Allerdings handelt es sich bei dieser Ausführungsform gemäß der Definition des Europäischen Arzneibuches um eine Tablette mit veränderter Wirkstofffreisetzung, in diesem Fall um eine sogenannte Retard-Tablette, bei der der Zerfall der Tablette bewusst verlangsamt ist und der Wirkstoff kontrolliert über einen Zeitraum von mehr als sechs Stunden freigesetzt wird.

Gegenüber den schnell-freisetzenden erfindungsgemäßen kompakten und Laktosefreien Tabletten und den in der EP2072054A1 beschriebenen schnell-freisetzenden großen und Laktose-haltigen Tabletten hat die in der WO2012/146592A1 (erteilt als EP2701688B1) beschriebene Retard-Tablette den Nachteil, dass der Wirkstoff langsamer in den Körper aufgenommen wird und später wirksam wird. Bisher wurde in Deutschland keine Retard-Tablette mit dem Wirkstoff Ginkgo-Blätterextrakt in den Markt eingeführt, weil das Freisetzungsverhalten dieser Tablette von allen anderen Produkten abweicht und daher die Wirksamkeit erst noch in teuren klinischen Prüfungen nachgewiesen werden muss.

Die spezielle, kontrollierte Wirkstofffreisetzung der Retard-Tablette wird durch die Wahl der Hilfsstoffe nach Art und Menge erreicht, d.h. durch die Verwendung einer geringen Menge von Hilfsstoffen, durch die Verwendung von wasserunlöslicher, wenig quellfähiger Ethylcellulose als Retardierungsmittel und den Verzicht von Zerfallsbeschleunigern wie Croscarmellose Natrium.

Die DE 20 2014 005 450 U1 beschreibt ein laktosefreies Arzneimittel, enthaltend einen Trockenextrakt aus Artischockenblättern. Die Ausführungsform ist eine feste pharmazeutische Darreichungsform in Form einer Hartkapsel oder einer Tablette. Zwar macht dieses Dokument Angaben zur qualitativen Zusammensetzung des laktosefreien Arzneimittels, aber keine Angaben, wie die sachgerechte Herstellung dieser Form erreicht wird und wie groß bzw. schwer die hergestellte Arzneiform ist.

Die Aufgabenstellung war die Bereitstellung eines Verfahrens zur Herstellung einer schnell zerfallenden Tablette mit einer Zerfallszeit von höchstens 15 Minuten zur peroralen Verabreichung eines Trockenextraktes aus den Blättern von Ginkgo biloba und mit einem Gesamtgewicht der Tablette zwischen 150 mg und 300 mg pro 100 mg des enthaltenen Ginkgoextrakts. Aufgabe war ferner die Bereitstellung schnell zerfallender Tabletten mit Trockenextrakt aus den Blättern von Ginkgo biloba mit kleinen Abmessungen, also guter Schluckbarkeit und, in einer bevorzugten Ausführungsform, ohne Laktose, also mit guter Verträglichkeit.

Der Trockenextrakt aus Ginkgo biloba-Blättern ist ein sehr feines, braun gefärbtes Pulver, das bei offener Lagerung sehr schnell Feuchte aus der Umgebung aufnimmt und zum Verkleben neigt. Bedingt durch die geringe Teilchengröße von mindestens 90 % < 100 µm und die schnelle und starke Feuchteaufnahme sind Mischungen von diesem Extrakt mit den üblichen pharmazeutischen Hilfsstoffen schlecht fließfähig und für die Herstellung von Tabletten durch direkte Komprimierung nur schlecht geeignet. Aus diesem Grund werden Hilfsstoffe benötigt, welche die Fließfähigkeit des Extraktes verbessern und die Herstellung einer Tablette durch Verpressung der Pulvermischung ermöglichen. Ein idealer Hilfsstoff zur Verbesserung der Fließfähigkeit und Verpressbarkeit ist Laktose-Monohydrat. Aus diesem Grund ist Laktose in nahezu allen hochdosierten Produkten mit Ginkgoblätter-Extrakt enthalten.

Überraschenderweise konnte jetzt durch Verringerung der spezifischen Oberfläche des Ginkgo-Extraktes durch Zumischen eines geringen Anteils der in der Tablette enthaltenen Hilfsstoffe und Verdichtung dieser Mischung mittels Kompaktierung und anschließende Zerkleinerung ein konzentriertes Granulat (Kompaktat) hergestellt werden (Schritt (a); geringer Anteil an Hilfsstoffen bedeutet in diesem Zusammenhang, dass die Kompaktatmasse das 1,14fache bis 1,57fache der Wirkstoffmasse beträgt). Dieses verdichtete Granulat ist so gut fließfähig, dass keine Laktose benötigt wird, um die für die Tablettenherstellung erforderlichen Fließeigenschaften zu erzielen (siehe Vergleichsbeispiel 1).

Durch Mischen dieses Granulates mit weiteren pharmazeutisch üblichen Hilfsstoffen, d.h. mikrokristalliner Cellulose (Bindemittel), Croscarmellose Natrium, Carboxymethylstärke Natrium, Crospovidone oder Natriumstärkeglycolat (Zerfallsbeschleuniger), hochdispersem Siliciumdioxid oder gefälltem Siliciumdioxid (Fließregulierungsmittel) und Magnesiumstearat, Stearinsäure, Behensäure, Natriumstearylfumarat, Glyceroldibehenat, Calciumbehenat oder Fumarsäure (Formentrennmittel) werden Tabletten erhalten (Schritt (b)), die trotz des hohen Wirkstoffanteils und des geringen Anteils an Hilfsstoffen eine sehr kurze Zerfallszeit aufweisen (siehe Tabelle 2), die sogar noch niedriger ist, als die der Tabletten aus Vergleichsbeispiel 1.

Gegenstand der Erfindung ist somit ein Verfahren zur Herstellung einer schnell zerfallenden Tablette mit einer Zerfallszeit von höchstens 15 Minuten zur peroralen Verabreichung eines Trockenextraktes aus den Blättern von Ginkgo biloba und mit einem Gesamtgewicht der Tablette zwischen 150 mg und 300 mg pro 100 mg des enthaltenen Ginkgoextrakts (entsprechend einem Gesamtgewicht zwischen 360 mg und 720 mg im Fall der 240 mg Ginkgoextrakt enthaltenden Tablette), umfassend
(a) einen ersten Verfahrensschritt, in dem der Ginkgoextrakt mit 6,94 mg bis 27,78 mg Bindemittel pro 100 mg des enthaltenen Ginkgoextrakts, mit 5,56 mg bis 22,22 mg Zerfallsbeschleuniger pro 100 mg des enthaltenen Ginkgoextrakts, mit 1,11 mg bis 4,44 mg Fließregulierungsmittel pro 100 mg des enthaltenen Ginkgoextrakts und mit 0,28 mg bis 1,11 mg Formentrennmittel pro 100 mg des enthaltenen Ginkgoextrakts gemischt und anschließend kompaktiert und zerkleinert wird, um ein konzentriertes Granulat (Kompaktat) zu erhalten, und
(b) einen zweiten Verfahrensschritt, in dem das in Schritt (a) erhaltene konzentrierte Granulat mit weiteren 22,78 mg bis 91,11 mg Bindemittel pro 100 mg des enthaltenen Ginkgoextrakts, mit weiteren 11,11 mg bis 44,44 mg Zerfallsbeschleuniger pro 100 mg des enthaltenen Ginkgoextrakts, mit weiteren 0,56 mg bis 2,22 mg Fließregulierungsmittel pro 100 mg des enthaltenen Ginkgoextrakts und mit weiteren 1,67 mg bis 6,67 mg Formentrennmittel pro 100 mg des enthaltenen Ginkgoextrakts gemischt und zu Tabletten gepresst wird,

wobei die Gesamtmenge an Hilfsstoffen in Schritt (a) 13,89 mg bis 55,56 mg pro 100 mg des enthaltenen Ginkgoextrakts und in Schritt (b) 36,11 mg bis 144,44 mg pro 100 mg des enthaltenen Ginkgoextrakts beträgt und
wobei das Bindemittel in Schritt (a) und in Schritt (b) mikrokristalline Cellulose ist, der Zerfallsbeschleuniger in Schritt (a) und in Schritt (b) unabhängig voneinander ausgewählt ist aus Croscarmellose Natrium, Carboxymethylstärke Natrium, Crospovidone oder Natriumstärkeglycolat, das Fließregulierungsmittel in Schritt (a) und in Schritt (b) unabhängig voneinander ausgewählt ist aus hochdispersem Siliciumdioxid oder gefälltem Siliciumdioxid und das Formentrennmittel in Schritt (a) und in Schritt (b) unabhängig voneinander ausgewählt ist aus Magnesiumstearat, Stearinsäure, Behensäure, Natriumstearylfumarat, Glyceroldibehenat, Calciumbehenat oder Fumarsäure.

Bevorzugt ist dabei ein Verfahren zur Herstellung einer schnell zerfallenden Tablette mit einer Zerfallszeit von höchstens 15 Minuten zur peroralen Verabreichung eines Trockenextraktes aus den Blättern von Ginkgo biloba und mit einem Gesamtgewicht der Tablette zwischen 160 mg und 200 mg pro 100 mg des enthaltenen Ginkgoextrakts (entsprechend einem Gesamtgewicht zwischen 384 mg und 480 mg im Fall der 240 mg Ginkgoextrakt enthaltenden Tablette), umfassend
(a) einen ersten Verfahrensschritt, in dem der Ginkgoextrakt mit 8,33 mg bis 13,89 mg Bindemittel pro 100 mg des enthaltenen Ginkgoextrakts, mit 6,67 mg bis 11,11 mg Zerfallsbeschleuniger pro 100 mg des enthaltenen Ginkgoextrakts, mit 1,33 mg bis 2,22 mg Fließregulierungsmittel pro 100 mg des enthaltenen Ginkgoextrakts und mit 0,33 mg bis 0,56 mg Formentrennmittel pro 100 mg des enthaltenen Ginkgoextrakts gemischt und anschließend kompaktiert und zerkleinert wird, um ein konzentriertes Granulat (Kompaktat) zu erhalten, und
(b) einen zweiten Verfahrensschritt, in dem das in Schritt (a) erhaltene konzentrierte Granulat mit weiteren 27,33 mg bis 45,56 mg Bindemittel pro 100 mg des enthaltenen Ginkgoextrakts, mit weiteren 13,33 mg bis 22,22 mg Zerfallsbeschleuniger pro 100 mg des enthaltenen Ginkgoextrakts, mit weiteren 0,67 mg bis 1,11 mg Fließregulierungsmittel pro 100 mg des enthaltenen Ginkgoextrakts und mit weiteren 2,00 mg bis 3,33 mg Formentrennmittel pro 100 mg des enthaltenen Ginkgoextrakts gemischt und zu Tabletten gepresst wird,

wobei die Gesamtmenge an Hilfsstoffen in Schritt (a) 16,67 mg bis 27,78 mg pro 100 mg des enthaltenen Ginkgoextrakts und in Schritt (b) 43,33 mg bis 72,22 mg pro 100 mg des enthaltenen Ginkgoextrakts beträgt und
wobei das Bindemittel in Schritt (a) und in Schritt (b) mikrokristalline Cellulose ist, der Zerfallsbeschleuniger in Schritt (a) und in Schritt (b) unabhängig voneinander ausgewählt ist aus Croscarmellose Natrium, Carboxymethylstärke Natrium, Crospovidone oder Natriumstärkeglycolat, das Fließregulierungsmittel in Schritt (a) und in Schritt (b) unabhängig voneinander ausgewählt ist aus hochdispersem Siliciumdioxid oder gefälltem Siliciumdioxid und das Formentrennmittel in Schritt (a) und in Schritt (b) unabhängig voneinander ausgewählt ist aus Magnesiumstearat, Stearinsäure, Behensäure, Natriumstearylfumarat, Glyceroldibehenat, Calciumbehenat oder Fumarsäure.

In einer bevorzugten Ausführungsform des vorstehenden Verfahrens ist der Zerfallsbeschleuniger in Schritt (a) und in Schritt (b) Croscarmellose Natrium, das Fließregulierungsmittel in Schritt (a) und in Schritt (b) gefälltes Siliciumdioxid und das Formentrennmittel in Schritt (a) und in Schritt (b) Magnesiumstearat.

In einer weiteren bevorzugten Ausführungsform des vorstehenden Verfahrens erfolgt das Kompaktieren in Schritt (a) durch Walzenkompaktierung.

In einer weiteren bevorzugten Ausführungsform des vorstehenden Verfahrens kommen außer Ginkgoextrakt keine weiteren Pflanzenextrakte oder sonstige Wirkstoffe und außer den genannten Hilfsstoffen keine weiteren Hilfsstoffe zum Einsatz.

Die Hilfsstoffe in Schritt (a) des vorstehend beschriebenen Verfahrens sind zusammengesetzt aus 45 Gew.-% bis 55 Gew.-% Bindemittel, 36 Gew.-% bis 44 Gew.-% Zerfallsbeschleuniger, 7 Gew.-% bis 9 Gew.-% Fließregulierungsmittel und 1,5 Gew.-% bis 2,5 Gew.-% Formentrennmittel. Die Hilfsstoffzusammensetzung in Schritt (b) beträgt 57 Gew.-% bis 69 Gew.-% Bindemittel, 28 Gew.-% bis 34 Gew.-% Zerfallsbeschleuniger, 1 Gew.-% bis 2 Gew.-% Fließregulierungsmittel und 4 Gew.-% bis 5 Gew.-% Formentrennmittel.

Gegenstand der Erfindung ist ferner eine schnell zerfallende Tablette mit einer Zerfallszeit von höchstens 15 Minuten zur peroralen Verabreichung eines Trockenextraktes aus den Blättern von Ginkgo biloba und mit einem Gesamtgewicht der Tablette zwischen 150 mg und 300 mg pro 100 mg des enthaltenen Ginkgoextrakts (entsprechend einem Gesamtgewicht zwischen 360 mg und 720 mg im Fall der 240 mg Ginkgoextrakt enthaltenden Tablette), hergestellt nach dem vorstehend beschriebenen Verfahren.

In einer bevorzugten Ausführungsform ist die Tablette dadurch gekennzeichnet, dass 80 bis 360 mg Trockenextrakt aus den Blättern von Ginkgo biloba enthalten sind.

In einer besonders bevorzugten Ausführungsform ist die Tablette dadurch gekennzeichnet, dass 220 bis 260 mg Trockenextrakt aus den Blättern von Ginkgo biloba enthalten sind.

In einer noch bevorzugteren Ausführungsform ist die Tablette dadurch gekennzeichnet, dass 240 mg Trockenextrakt aus den Blättern von Ginkgo biloba enthalten sind.

Bevorzugt enthalten die vorstehend genannten Tabletten einen Trockenextrakt aus den Blättern von Ginkgo biloba enthaltend 22,0 bis 27,0 % Flavonoide berechnet als Flavonglycoside, 2,6 bis 3,2 % Bilobalid, 2,8 bis 3,4 % Ginkgolide A, B und C und höchstens 5 ppm Ginkgolsäuren. Die Prozentangaben beziehen sich auf das Gewicht (Gew.-%).

In einer weiteren bevorzugten Ausführungsform enthalten die vorstehend beschriebenen Tabletten keine Laktose.

In einer weiteren bevorzugten Ausführungsform sind die vorstehend beschriebenen Tabletten zusätzlich mit einem Film überzogen und weisen eine Zerfallszeit von höchstens 30 Minuten auf.

Darüber hinaus ist Gegenstand der Erfindung auch eine schnell zerfallende Tablette mit einer Zerfallszeit von höchstens 15 Minuten zur peroralen Verabreichung eines Trockenextraktes aus den Blättern von Ginkgo biloba, dadurch gekennzeichnet, dass das Gesamtgewicht der Tablette zwischen 150 mg und 300 mg pro 100 mg des enthaltenen Ginkgoextrakts (entsprechend einem Gesamtgewicht zwischen 360 mg und 720 mg im Fall der 240 mg Ginkgoextrakt enthaltenden Tablette) beträgt, wobei die Tablette neben dem Ginkgoextrakt mikrokristalline Cellulose als Bindemittel, einen Zerfallsbeschleuniger ausgewählt aus Croscarmellose Natrium, Carboxymethylstärke Natrium, Crospovidone oder Natriumstärkeglycolat, ein Fließregulierungsmittel ausgewählt aus hochdispersem Siliciumdioxid oder gefälltem Siliciumdioxid und ein Formentrennmittel ausgewählt aus Magnesiumstearat, Stearinsäure, Behensäure, Natriumstearylfumarat, Glyceroldibehenat, Calciumbehenat oder Fumarsäure enthält, wobei keine Laktose enthalten ist, und außer Ginkgoextrakt keine weiteren Pflanzenextrakte oder sonstige Wirkstoffe und außer den genannten Hilfsstoffen keine weiteren Hilfsstoffe enthalten sind.

Bevorzugt beträgt dabei das Gesamtgewicht der Tablette zwischen 160 mg und 200 mg pro 100 mg des enthaltenen Ginkgoextrakts (entsprechend einem Gesamtgewicht zwischen 384 mg und 480 mg im Fall der 240 mg Ginkgoextrakt enthaltenden Tablette).

Besonders bevorzugt beträgt dabei das Gesamtgewicht der Tablette 175 mg pro 100 mg des enthaltenen Ginkgoextrakts (entsprechend einem Gesamtgewicht von 420 mg im Fall der 240 mg Ginkgoextrakt enthaltenden Tablette).

In einer weiteren bevorzugten Ausführungsform sind in der Tablette 80 bis 360 mg Trockenextrakt aus den Blättern von Ginkgo biloba enthalten.

Besonders bevorzugt sind dabei in der Tablette 220 bis 260 mg Trockenextrakt aus den Blättern von Ginkgo biloba enthalten.

Ganz besonders bevorzugt sind dabei in der Tablette 240 mg Trockenextrakt aus den Blättern von Ginkgo biloba enthalten.

Bevorzugt enthalten die vorstehend genannten Tabletten einen Trockenextrakt aus den Blättern von Ginkgo biloba enthaltend 22,0 bis 27,0 % Flavonoide berechnet als Flavonglycoside, 2,6 bis 3,2 % Bilobalid, 2,8 bis 3,4 % Ginkgolide A, B und C und höchstens 5 ppm Ginkgolsäuren. Die Prozentangaben beziehen sich auf das Gewicht (Gew.-%).

In einer weiteren bevorzugten Ausführungsform enthalten die vorstehend beschriebenen Tabletten keine Laktose.

In einer weiteren bevorzugten Ausführungsform enthalten die vorstehend beschriebenen Tabletten außer Ginkgoextrakt keine weiteren Pflanzenextrakte oder sonstige Wirkstoffe und außer den genannten Hilfsstoffen keine weiteren Hilfsstoffe.

Ferner enthalten die vorstehend beschriebenen Tabletten bevorzugt mikrokristalline Cellulose, Croscarmellose Natrium, gefälltes Siliciumdioxid und Magnesiumstearat als Hilfsstoffe.

In einer weiteren bevorzugten Ausführungsform sind die vorstehend beschriebenen

Tabletten zusätzlich mit einem Film überzogen und weisen eine Zerfallszeit von höchstens 30 Minuten auf.

Die Mengenangaben in vorstehender Beschreibung, in den Beispielen und in den Ansprüchen sind durch proportionale Umrechnung der Zusammensetzung in Beispiel 2 und durch den Bezug auf 100 mg Ginkgoextrakt entstanden und auf zwei Nachkommastellen gerundet. Mögliche Ungenauigkeiten bei Summenbildungen sind daher nicht auszuschließen.

### Erfindungsgemäßes Beispiel 1: Kompakte, laktosefreie Tabletten ohne Überzug mit 240 mg Trockenextrakt aus Ginkgo biloba Blättern mit einem Verhältnis der Tablettenmasse zur enthaltenen Wirkstoffmasse von 1,5

| | Bestandteil | Menge pro Tablette [mg] | | Berechnet auf 100 mg des enthaltenen Ginkgoextrakts [mg] | |
|---|---|---|---|---|---|
| 1. | Ginkgoblätter-Extrakt (EGb^{®} 761) | 240,00 | | 100,00 | |
| 2. | Cellulose, mikrokristallin | 16,67 | | 6,94 | |
| 3. | Croscarmellose Natrium | 13,33 | | 5,56 | |
| 4. | Siliciumdioxid, gefällt | 2,67 | | 1,11 | |
| 5. | Magnesiumstearat | 0,67 | | 0,28 | |
| 2. - 5. | Summe der Hilfsstoffe in Schritt (a) | 33,34 | | 13,89 | |
| 1.-5. | Kompaktat | 273,34 | | 113,89 | |
| 6. | Cellulose, mikrokristallin | 54,66 | | 22,78 | |
| 7. | Croscarmellose Natrium | 26,67 | | 11,11 | |
| 8. | Siliciumdioxid, gefällt | 1,33 | | 0,56 | |
| 9. | Magnesiumstearat | 4,00 | | 1,67 | |
| 6. - 9. | Summe der Hilfsstoffe in Schritt (b) | 86,66 | | 36,11 | |
| 1.-9. | Tablette | 360,00 | | 150,00 | |

Zur Herstellung der erfindungsgemäßen Tabletten gemäß Beispiel 1 werden 480 g Ginkgoblätter-Extrakt mit 33,34 g mikrokristalliner Cellulose, 26,66 g Croscarmellose Natrium, 5,34 g gefälltem Siliciumdioxid und 1,34 g Magnesiumstearat gemischt und mit einem Walzenkompaktor zu einem konzentrierten Granulat (Kompaktat) verarbeitet (Schritt (a)). Zur Herstellung von 1600 Tabletten werden 437,34 g des so erhaltenen Granulats mit 87,46 g mikrokristalliner Cellulose, 42,67 g Croscarmellose Natrium, 2,13 g gefälltem Siliciumdioxid und 6,40 g Magnesiumstearat gemischt und auf einer Rundläufertablettenpresse zu Tabletten mit einer Masse von jeweils 360 mg verpresst (Schritt (b)).

### Erfindungsgemäßes Beispiel 2: Kompakte, laktosefreie Tabletten ohne Überzug mit 240 mg Trockenextrakt aus Ginkgo biloba Blättern mit einem Verhältnis der Tablettenmasse zur enthaltenen Wirkstoffmasse von 1,75

| | Bestandteil | Menge pro Tablette [mg] | | Berechnet auf 100 mg des enthaltenen Ginkgoextrakts [mg] | |
|---|---|---|---|---|---|
| 1. | Ginkgoblätter-Extrakt (EGb^{®} 761) | 240,00 | | 100,00 | |
| 2. | Cellulose, mikrokristallin | 25,00 | | 10,42 | |
| 3. | Croscarmellose Natrium | 20,00 | | 8,33 | |
| 4. | Siliciumdioxid, gefällt | 4,00 | | 1,67 | |
| 5. | Magnesiumstearat | 1,00 | | 0,42 | |
| 2. - 5. | Summe der Hilfsstoffe in Schritt (a) | 50,00 | | 20,83 | |
| 1.-5. | Kompaktat | 290,00 | | 120,83 | |
| 6. | Cellulose, mikrokristallin | 82,00 | | 34,17 | |
| 7. | Croscarmellose Natrium | 40,00 | | 16,67 | |
| 8. | Siliciumdioxid, gefällt | 2,00 | | 0,83 | |
| 9. | Magnesiumstearat | 6,00 | | 2,50 | |
| 6. - 9. | Summe der Hilfsstoffe in Schritt (b) | 130,00 | | 54,17 | |
| 1.-9. | Tablette | 420,00 | | 175,00 | |

Zur Herstellung von 100.000 Stück der erfindungsgemäßen Tabletten gemäß Beispiel 2 werden 24,00 kg Ginkgoblätter-Extrakt mit 2,50 kg mikrokristalliner Cellulose, 2,00 kg Croscarmellose Natrium, 0,40 kg gefälltem Siliciumdioxid und 0,10 kg Magnesiumstearat gemischt und mit einem Walzenkompaktor zu einem konzentrierten Granulat (Kompaktat) verarbeitet (Schritt (a)). Das Granulat wird mit 8,20 kg mikrokristalliner Cellulose, 4,00 kg Croscarmellose Natrium, 0,20 kg gefälltem Siliciumdioxid und 0,60 kg Magnesiumstearat gemischt und auf einer Rundläufertablettenpresse zu Tabletten mit einer Masse von jeweils 420 mg verpresst (Schritt (b)).

### Erfindungsgemäßes Beispiel 3: Kompakte, laktosefreie Tabletten ohne Überzug mit 240 mg Trockenextrakt aus Ginkgo biloba Blättern mit einem Verhältnis der Tablettenmasse zur enthaltenen Wirkstoffmasse von 3,0

| | Bestandteil | Menge pro Tablette [mg] | | Berechnet auf 100 mg des enthaltenen Ginkgoextrakts [mg] | |
|---|---|---|---|---|---|
| 1. | Ginkgoblätter-Extrakt (EGb^{®} 761) | 240,00 | | 100,00 | |
| 2. | Cellulose, mikrokristallin | 66,67 | | 27,78 | |
| 3. | Croscarmellose Natrium | 53,33 | | 22,22 | |
| 4. | Siliciumdioxid, gefällt | 10,67 | | 4,44 | |
| 5. | Magnesiumstearat | 2,67 | | 1,11 | |
| 2. - 5. | Summe der Hilfsstoffe in Schritt (a) | 133,34 | | 55,56 | |
| 1.-5. | Kompaktat | 373,34 | | 155,56 | |
| 6. | Cellulose, mikrokristallin | 218,66 | | 91,11 | |
| 7. | Croscarmellose Natrium | 106,67 | | 44,44 | |
| 8. | Siliciumdioxid, gefällt | 5,33 | | 2,22 | |
| 9. | Magnesiumstearat | 16,00 | | 6,67 | |
| 6. - 9. | Summe der Hilfsstoffe in Schritt (b) | 346,66 | | 144,44 | |
| 1.-9. | Tablette | 720,00 | | 300,00 | |

Zur Herstellung der erfindungsgemäßen Tabletten gemäß Beispiel 3 werden 480 g Ginkgoblätter-Extrakt mit 133,34 g mikrokristalliner Cellulose, 106,66 g Croscarmellose Natrium, 21,34 g gefälltem Siliciumdioxid und 5,34 g Magnesiumstearat gemischt und mit einem Walzenkompaktor zu einem konzentrierten Granulat (Kompaktat) verarbeitet (Schritt (a)). Zur Herstellung von 1600 Tabletten werden 597,34 g des so erhaltenen Granulats mit 349,66 g mikrokristalliner Cellulose, 170,67 g Croscarmellose Natrium, 8,53 g gefälltem Siliciumdioxid und 25,60 g Magnesiumstearat gemischt und auf einer Rundläufertablettenpresse zu Tabletten mit einer Masse von jeweils 720 mg verpresst (Schritt (b)).

### Erfindungsgemäßes Beispiel 4: Kompakte, laktosefreie Tablette mit Überzug mit 240 mg Trockenextrakt aus Ginkgo biloba Blättern

Die erfindungsmäßen laktosefreien, kompakten Tabletten aus Beispiel 2 können mit einem dünnen Farbüberzug versehen werden, der den Zerfall der Tabletten und damit die Freisetzung des Wirkstoffes kaum verzögert (vgl. Tabelle 2).

| | Bestandteil | Massenanteil in mg pro Filmtablette | |
|---|---|---|---|
| 1. | Ginkgoblätter-Extrakt (EGb^{®} 761) | 240,00 | |
| 2. | Cellulose, mikrokristallin | 107,00 | |
| 3. | Croscarmellose Natrium | 60,00 | |
| 4. | Siliciumdioxid, gefällt | 6,00 | |
| 5. | Magnesiumstearat | 7,00 | |
| | Zwischensumme Tablettengewicht ohne Überzug | 420,00 | |
| 6. | Hypromellose | 4,80 | |
| 7. | Mikrokristalline Cellulose | 2,00 | |
| 8. | Glycerol, wasserfrei | 0,70 | |
| 9. | Eisenoxid E172 | 3,00 | |
| 10. | Talkum | 1,50 | |
| | Zwischensumme Filmüberzugsgewicht | 12,00 | |
| | Gesamtsumme Filmtablettengewicht | 434,00 | |

### Nicht-erfindungsgemäße Vergleichsbeispiele:

### Vergleichsbeispiel 1 aus EP 2072054A1

Die EP2072054A1 beschreibt die Verwendung eines Extraktes aus Blättern von Ginkgo biloba in der bevorzugten Ausführungsform einer Tablette mit einer Masse von 800 mg, davon 160 mg Laktose-Monohydrat. Zur Herstellung (Absatz [0033]) der Tablette gemäß erfindungsgemäßem Beispiel 3 der EP 2072054A1 wird der Extrakt mit den in nachfolgender Tabelle genannten Hilfsstoffen gemischt und ohne weitere Verfahrensschritte direkt zu Tabletten gepresst. Bei der beschriebenen Tablette handelt es sich um eine Tablette mit schneller Wirkstofffreisetzung (EP2072054A1, Anspruch 15 und Tabelle zu erfindungsgemäßem Beispiel 1). Diese Ausführungsform ist deutlich größer und schwerer als die erfindungsgemäße Tablette und enthält Laktose Monohydrat.

| | Bestandteil | Menge pro Tablette [mg] | | Berechnet auf 100 mg des enthaltenen Ginkgoextrakts [mg] | |
|---|---|---|---|---|---|
| 1. | Ginkgoblätter-Extrakt EGb^{®} 761 | 240,00 | | 100,00 | |
| 2. | Cellulose, mikrokristallin | 290,00 | | 120,83 | |
| 3. | Laktosemonohydrat | 160,00 | | 66,67 | |
| 4. | Maisstärke | 50,00 | | 20,83 | |
| 5. | Croscarmellose Natrium | 40,00 | | 16,67 | |
| 6. | Siliciumdioxid, hochdispers | 10,00 | | 4,17 | |
| 7. | Magnesiumstearat | 10,00 | | 4,17 | |
| | Tablettengewicht | 800,00 | | 333,33 | |

### Vergleichsbeispiel 2 aus EP 2701688B1

Die WO2012/146592A1 (erteilt als EP2701688B1) beschreibt eine Tablette mit 240 mg Trockenextrakt aus Ginkgo biloba Blättern mit kontrollierter Freisetzung und deren Herstellung. Die Herstellung der Tablette erfolgt wie im erfindungsgemäßen Beispiel 1 der WO2012/146592A1 beschrieben. Die Zusammensetzung ist dabei der nachstehenden Tabelle zu entnehmen. Diese Tablette ist laktosefrei, enthält sehr wenig Hilfsstoffe und ist damit sehr kompakt. Allerdings handelt es sich bei dieser Ausführungsform gemäß der Definition des Europäischen Arzneibuches um eine Tablette mit veränderter Wirkstofffreisetzung, in diesem Fall um eine sogenannte Retard-Tablette, bei der der Zerfall der Tablette bewusst verlangsamt ist und der Wirkstoff kontrolliert über einen Zeitraum von mehr als sechs Stunden freigesetzt wird ( siehe Tabelle zu erfindungsgemäßem Beispiel 1).

| | Bestandteil | Menge pro Tablette [mg] | Berechnet auf 100 mg des enthaltenen Ginkgoextrakts [mg] | |
|---|---|---|---|---|
| 1. | Ginkgoblätter-Extrakt EGb^{®} 761 | 240,00 | 100,00 | |
| 2. | Ethylcellulose | 170,00 | 70,83 | |
| 3. | Siliciumdioxid, hochdispers | 2,00 | 0,83 | |
| 4. | Magnesiumstearat | 8,00 | 3,33 | |
| | Tablettengewicht | 420,00 | 175,00 | |

### Vergleich der Zerfallszeiten und Abmessungen

Nach den Vorgaben des Europäischen Arzneibuches Ph.Eur. 2.9.1 wurden die Zerfallszeiten der erfindungsgemäßen kompakten Tabletten gemäß den Beispielen 1 bis 4 und den Tabletten von Vergleichsbeispiel 1 (große, schnell-zerfallende Tablette mit Laktose) und Vergleichsbeispiel 2 (kompakte Retard-Tablette) bestimmt.

**Tabelle 2: Zerfallszeiten und Abmessungen der Tabletten aus den erfindungsgemäßen Beispielen 1 bis 4 und den Vergleichsbeispielen 1 und 2**

| Ausführungsform | Chargen-Bezeichnung | TM/WM* | Zerfallszeit, n = 6 [min] | Abmessungen Länge/Breite/Dicke [mm] |
|---|---|---|---|---|
| Erfindungsgemäße kompakte Tablette (ohne Überzug) gemäß Beispiel 1 | 201804 | 1,50 | 5:14-7:00 | 14,46/7,56/4,13 |
| Erfindungsgemäße kompakte Tablette (ohne Überzug) gemäß Beispiel 2 | P201602 | 1,75 | 7:18-9:32 | 14,08/7,07/5,18 |
| Erfindungsgemäße kompakte Tablette (ohne Überzug) gemäß Beispiel 3 | 201805 | 3,00 | 4:12 - 5:04 | 17,14/8,08/6,97 |
| Erfindungsgemäße kompakte Filmtablette (mit Überzug) gemäß Beispiel 4 | P201602 | 1,75 (ohne Überzug) | 9:14 - 11:16 | 14,06/7,09/5,28 |
| Tablette (ohne Überzug) gemäß Vergleichsbeispiel 1 | P201301 | 3,33 | 10:20 - 13:00 | 19,15/8,07/5,82 |
| Retard-Tablette (ohne Überzug) gemäß Vergleichsbeispiel 2 | 201401 | 1,75 | >30 min | runde Tablette (11 mm) |

| | | | | |
|---|---|---|---|---|
| *TM/WM = Verhältnis Tablettenmasse/Wirkstoffmasse | | | | |

Die erfindungsgemäßen Tabletten haben trotz eines hohen Wirkstoffanteils bzw. der niedrigen Tablettenmasse im Verhältnis zur enthaltenen Wirkstoffmasse eine kurze Zerfallszeit von unter 15 Minuten (ohne Überzug, Beispiele 1 bis 3) bzw. von unter 30 Minuten (mit Überzug, Beispiel 4) und entsprechen damit den Vorgaben des Europäischen Arzneibuchs.

Dabei sind die erfindungsgemäßen Tabletten der Beispiele 1 bis 4 bei gleichem Wirkstoffgehalt kleiner als die Tabletten des Vergleichsbeispiels 1.

## Patentansprüche

1. Verfahren zur Herstellung einer schnell zerfallenden Tablette mit einer Zerfallszeit von höchstens 15 Minuten zur peroralen Verabreichung eines Trockenextraktes aus den Blättern von Ginkgo biloba und mit einem Gesamtgewicht der Tablette zwischen 150 mg und 300 mg pro 100 mg des enthaltenen Ginkgoextrakts, umfassend
(a) einen ersten Verfahrensschritt, in dem der Ginkgoextrakt mit 6,94 mg bis 27,78 mg Bindemittel pro 100 mg des enthaltenen Ginkgoextrakts, mit 5,56 mg bis 22,22 mg Zerfallsbeschleuniger pro 100 mg des enthaltenen Ginkgoextrakts, mit 1,11 mg bis 4,44 mg Fließregulierungsmittel pro 100 mg des enthaltenen Ginkgoextrakts und mit 0,28 mg bis 1,11 mg Formentrennmittel pro 100 mg des enthaltenen Ginkgoextrakts gemischt und anschließend kompaktiert und zerkleinert wird, um ein konzentriertes Granulat zu erhalten, und
(b) einen zweiten Verfahrensschritt, in dem das in Schritt (a) erhaltene konzentrierte Granulat mit weiteren 22,78 mg bis 91,11 mg Bindemittel pro 100 mg des enthaltenen Ginkgoextrakts, mit weiteren 11,11 mg bis 44,44 mg Zerfallsbeschleuniger pro 100 mg des enthaltenen Ginkgoextrakts, mit weiteren 0,56 mg bis 2,22 mg Fließregulierungsmittel pro 100 mg des enthaltenen Ginkgoextrakts und mit weiteren 1,67 mg bis 6,67 mg Formentrennmittel pro 100 mg des enthaltenen Ginkgoextrakts gemischt und zu Tabletten gepresst wird,
wobei die Gesamtmenge an Hilfsstoffen in Schritt (a) 13,89 mg bis 55,56 mg pro 100 mg des enthaltenen Ginkgoextrakts und in Schritt (b) 36,11 mg bis 144,44 mg pro 100 mg des enthaltenen Ginkgoextrakts beträgt und
wobei das Bindemittel in Schritt (a) und in Schritt (b) mikrokristalline Cellulose ist, der Zerfallsbeschleuniger in Schritt (a) und in Schritt (b) unabhängig voneinander ausgewählt ist aus Croscarmellose Natrium, Carboxymethylstärke Natrium, Crospovidone oder Natriumstärkeglycolat, das Fließregulierungsmittel in Schritt (a) und in Schritt (b) unabhängig voneinander ausgewählt ist aus hochdispersem Siliciumdioxid oder gefälltem Siliciumdioxid und das Formentrennmittel in Schritt (a) und in Schritt (b) unabhängig voneinander ausgewählt ist aus Magnesiumstearat, Stearinsäure, Behensäure, Natriumstearylfumarat, Glyceroldibehenat, Calciumbehenat oder Fumarsäure.

2. Verfahren nach Anspruch 1 zur Herstellung einer schnell zerfallenden Tablette mit einer Zerfallszeit von höchstens 15 Minuten zur peroralen Verabreichung eines Trockenextraktes aus den Blättern von Ginkgo biloba und mit einem Gesamtgewicht der Tablette zwischen 160 mg und 200 mg pro 100 mg des enthaltenen Ginkgoextrakts, **dadurch gekennzeichnet, dass**
(a) der Ginkgoextrakt in einem ersten Verfahrensschritt mit 8,33 mg bis 13,89 mg Bindemittel pro 100 mg des enthaltenen Ginkgoextrakts, mit 6,67 mg bis 11,11 mg Zerfallsbeschleuniger pro 100 mg des enthaltenen Ginkgoextrakts, mit 1,33 mg bis 2,22 mg Fließregulierungsmittel pro 100 mg des enthaltenen Ginkgoextrakts und mit 0,33 mg bis 0,56 mg Formentrennmittel pro 100 mg des enthaltenen Ginkgoextrakts gemischt und anschließend kompaktiert und zerkleinert wird, um ein konzentriertes Granulat zu erhalten, und
(b) das in Schritt (a) erhaltene konzentrierte Granulat in einem zweiten Verfahrensschritt mit weiteren 27,33 mg bis 45,56 mg Bindemittel pro 100 mg des enthaltenen Ginkgoextrakts, mit weiteren 13,33 mg bis 22,22 mg Zerfallsbeschleuniger pro 100 mg des enthaltenen Ginkgoextrakts, mit weiteren 0,67 mg bis 1,11 mg Fließregulierungsmittel pro 100 mg des enthaltenen Ginkgoextrakts und mit weiteren 2,00 mg bis 3,33 mg Formentrennmittel pro 100 mg des enthaltenen Ginkgoextrakts gemischt und zu Tabletten gepresst wird,
wobei die Gesamtmenge an Hilfsstoffen in Schritt (a) 16,67 mg bis 27,78 mg pro 100 mg des enthaltenen Ginkgoextrakts und in Schritt (b) 43,33 mg bis 72,22 mg des enthaltenen Ginkgoextrakts beträgt und
wobei das Bindemittel in Schritt (a) und in Schritt (b) mikrokristalline Cellulose ist, der Zerfallsbeschleuniger in Schritt (a) und in Schritt (b) unabhängig voneinander ausgewählt ist aus Croscarmellose Natrium, Carboxymethylstärke Natrium, Crospovidone oder Natriumstärkeglycolat, das Fließregulierungsmittel in Schritt (a) und in Schritt (b) unabhängig voneinander ausgewählt ist aus hochdispersem Siliciumdioxid oder gefälltem Siliciumdioxid und das Formentrennmittel in Schritt (a) und in Schritt (b) unabhängig voneinander ausgewählt ist aus Magnesiumstearat, Stearinsäure, Behensäure, Natriumstearylfumarat, Glyceroldibehenat, Calciumbehenat oder Fumarsäure.

3. Verfahren nach einem der Ansprüche 1 oder 2, wobei der Zerfallsbeschleuniger in Schritt (a) und in Schritt (b) Croscarmellose Natrium ist, das Fließregulierungsmittel in Schritt (a) und in Schritt (b) gefälltes Siliciumdioxid ist und das Formentrennmittel in Schritt (a) und in Schritt (b) Magnesiumstearat ist.

4. Verfahren nach einem der Ansprüche 1 bis 3, wobei das Kompaktieren in Schritt (a) durch Walzenkompaktierung erfolgt.

5. Verfahren nach einem der Ansprüche 1 bis 4, wobei außer Ginkgoextrakt keine weiteren Pflanzenextrakte oder sonstige Wirkstoffe und außer den genannten Hilfsstoffen keine weiteren Hilfsstoffe zum Einsatz kommen.

6. Schnell zerfallende Tablette mit einer Zerfallszeit von höchstens 15 Minuten zur peroralen Verabreichung eines Trockenextraktes aus den Blättern von Ginkgo biloba und mit einem Gesamtgewicht der Tablette zwischen 150 mg und 300 mg pro 100 mg des enthaltenen Ginkgoextrakts, hergestellt nach einem Verfahren nach einem der Ansprüche 1 bis 5.

7. Schnell zerfallende Tablette nach Anspruch 6, wobei die Tablette neben dem Ginkgoextrakt mikrokristalline Cellulose als Bindemittel, einen Zerfallsbeschleuniger ausgewählt aus Croscarmellose Natrium, Carboxymethylstärke Natrium, Crospovidone oder Natriumstärkeglycolat, ein Fließregulierungsmittel ausgewählt aus hochdispersem Siliciumdioxid oder gefälltem Siliciumdioxid und ein Formentrennmittel ausgewählt aus Magnesiumstearat, Stearinsäure, Behensäure, Natriumstearylfumarat, Glyceroldibehenat, Calciumbehenat oder Fumarsäure enthält, wobei keine Laktose enthalten ist, und außer Ginkgoextrakt keine weiteren Pflanzenextrakte oder sonstige Wirkstoffe und außer den genannten Hilfsstoffen keine weiteren Hilfsstoffe enthalten sind.

8. Schnell zerfallende Tablette mit einer Zerfallszeit von höchstens 15 Minuten zur peroralen Verabreichung eines Trockenextraktes aus den Blättern von Ginkgo biloba, **dadurch gekennzeichnet, dass** das Gesamtgewicht der Tablette zwischen 150 mg und 300 mg pro 100 mg des enthaltenen Ginkgoextrakts beträgt, wobei die Tablette neben dem Ginkgoextrakt mikrokristalline Cellulose als Bindemittel, einen Zerfallsbeschleuniger ausgewählt aus Croscarmellose Natrium, Carboxymethylstärke Natrium, Crospovidone oder Natriumstärkeglycolat, ein Fließregulierungsmittel ausgewählt aus hochdispersem Siliciumdioxid oder gefälltem Siliciumdioxid und ein Formentrennmittel ausgewählt aus Magnesiumstearat, Stearinsäure, Behensäure, Natriumstearylfumarat, Glyceroldibehenat, Calciumbehenat oder Fumarsäure enthält, wobei keine Laktose enthalten ist, und außer Ginkgoextrakt keine weiteren Pflanzenextrakte oder sonstige Wirkstoffe und außer den genannten Hilfsstoffen keine weiteren Hilfsstoffe enthalten sind.

9. Tablette nach Anspruch 6, 7 oder 8, **dadurch gekennzeichnet, dass** das Gesamtgewicht der Tablette zwischen 160 mg und 200 mg pro 100 mg des enthaltenen Ginkgoextrakts beträgt.

10. Tablette nach einem der Ansprüche 6 bis 9, **dadurch gekennzeichnet, dass** das Gesamtgewicht der Tablette 175 mg pro 100 mg des enthaltenen Ginkgoextrakts beträgt.

11. Tablette nach einem der Ansprüche 6 bis 10, **dadurch gekennzeichnet, dass** 80 bis 360 mg Trockenextrakt aus den Blättern von Ginkgo biloba enthalten sind.

12. Tablette nach einem der Ansprüche 6 bis 11, **dadurch gekennzeichnet, dass** 220 bis 260 mg Trockenextrakt aus den Blättern von Ginkgo biloba enthalten sind.

13. Tablette nach einem der Ansprüche 6 bis 12, **dadurch gekennzeichnet, dass** 240 mg Trockenextrakt aus den Blättern von Ginkgo biloba enthalten sind.

14. Tablette nach einem der Ansprüche 6 bis 13, **dadurch gekennzeichnet, dass** der Trockenextrakt aus den Blättern von Ginkgo biloba 22,0 bis 27,0 % Flavonoide berechnet als Flavonglycoside, 2,6 bis 3,2 % Bilobalid, 2,8 bis 3,4 % Ginkgolide A, B und C und höchstens 5 ppm Ginkgolsäuren enthält.

15. Tablette nach Anspruch 6, **dadurch gekennzeichnet, dass** keine Laktose enthalten ist.

16. Tablette nach Anspruch 6, wobei außer Ginkgoextrakt keine weiteren Pflanzenextrakte oder sonstige Wirkstoffe und außer den genannten Hilfsstoffen keine weiteren Hilfsstoffe enthalten sind.

17. Tablette nach einem der Ansprüche 6 oder 7 bis 16, **dadurch gekennzeichnet, dass** die Tablette mikrokristalline Cellulose, Croscarmellose Natrium, gefälltes Siliciumdioxid und Magnesiumstearat enthält.

18. Tablette nach einem der Ansprüche 6 oder 7 bis 17, **dadurch gekennzeichnet, dass** die Tablette zusätzlich mit einem Film überzogen ist und eine Zerfallszeit von höchstens 30 Minuten aufweist.

## Claims

1. A process for the preparation of a rapidly disintegrating tablet having a disintegration time of at most 15 minutes for the peroral administration of a dry extract from the leaves of Ginkgo biloba and having a total weight of the tablet between 150 mg and 300 mg per 100 mg of the contained ginkgo extract, comprising
(a) a first process step in which the ginkgo extract is mixed with 6.94 mg to 27.78 mg of binder per 100 mg of ginkgo extract contained, with 5.56 mg to 22.22 mg of disintegration accelerator per 100 mg of ginkgo extract contained, with 1.11 mg to 4.44 mg of flow regulating agent per 100 mg of ginkgo extract contained, and with 0.28 mg to 1.11 mg of mold release agent per 100 mg of ginkgo extract contained, and then compacted and comminuted to obtain concentrated granules, and
(b) a second process step in which the concentrated granules obtained in step (a) are mixed with further 22.78 mg to 91.11 mg of binder per 100 mg of ginkgo extract contained, with further 11.11 mg to 44.44 mg of disintegration accelerator per 100 mg of ginkgo extract contained, with further 0.56 mg to 2.22 mg of flow regulating agent per 100 mg of the ginkgo extract contained and with further 1.67 mg to 6.67 mg of mold release agent per 100 mg of the ginkgo extract contained and pressed into tablets,
wherein the total amount of excipients in step (a) is from 13.89 mg to 55.56 mg per 100 mg of ginkgo extract contained and in step (b) is from 36.11 mg to 144.44 mg per 100 mg of ginkgo extract contained; and
wherein the binder in step (a) and in step (b) is microcrystalline cellulose, the disintegration accelerator in step (a) and in step (b) is independently selected from croscarmellose sodium, carboxymethyl starch sodium, crospovidone or sodium starch glycolate, the flow control agent in step (a) and in step (b) is independently selected from highly dispersed silica or precipitated silica, and the mold release agent in step (a) and in step (b) is independently selected from magnesium stearate, stearic acid, behenic acid, sodium stearyl fumarate, glycerol dibehenate, calcium behenate or fumaric acid.

2. The process according to claim 1 for the preparation of a rapidly disintegrating tablet having a disintegration time of at most 15 minutes for the peroral administration of a dry extract from the leaves of Ginkgo biloba and having a total weight of the tablet of between 160 mg and 200 mg per 100 mg of ginkgo extract contained, **characterized in that**
(a) the ginkgo extract is mixed in a first process step with 8.33 mg to 13.89 mg of binder per 100 mg of ginkgo extract contained, with 6.67 mg to 11.11 mg of disintegration accelerator per 100 mg of ginkgo extract contained, with 1.33 mg to 2,22 mg of flow regulating agent per 100 mg of ginkgo extract contained, and with 0.33 mg to 0.56 mg of mold release agent per 100 mg of ginkgo extract contained, and then compacted and comminuted to obtain concentrated granules, and
(b) in a second process step, the concentrated granules obtained in step (a) are mixed with a further 27.33 mg to 45.56 mg of binder per 100 mg of ginkgo extract contained, with a further 13.33 mg to 22.22 mg of disintegration accelerator per 100 mg of ginkgo extract contained, with further 0.67 mg to 1.11 mg of flow regulating agent per 100 mg of the ginkgo extract contained and with further 2.00 mg to 3.33 mg of mold release agent per 100 mg of the ginkgo extract contained and pressed into tablets,
wherein the total amount of excipients in step (a) is 16.67 mg to 27.78 mg per 100 mg of ginkgo extract contained and in step (b) is 43.33 mg to 72.22 mg of ginkgo extract contained, and
wherein the binder in step (a) and in step (b) is microcrystalline cellulose, the disintegration accelerator in step (a) and in step (b) is independently selected from croscarmellose sodium, carboxymethyl starch sodium, crospovidone or sodium starch glycolate, the flow control agent in step (a) and in step (b) is independently selected from highly dispersed silica or precipitated silica, and the mold release agent in step (a) and in step (b) is independently selected from magnesium stearate, stearic acid, behenic acid, sodium stearyl fumarate, glycerol dibehenate, calcium behenate or fumaric acid.

3. The process of any one of claims 1 or 2, wherein the disintegration accelerator in step (a) and in step (b) is croscarmellose sodium, the flow regulating agent in step (a) and in step (b) is precipitated silica, and the mold release agent in step (a) and in step (b) is magnesium stearate.

4. The process according to any one of claims 1 to 3,
wherein the compacting in step (a) is performed by roller compacting.

5. The process according to any one of claims 1 to 4,
wherein, apart from ginkgo extract, no other plant extracts or other active ingredients and, apart from the excipients mentioned, no other excipients are used.

6. A rapidly disintegrating tablet having a disintegration time of at most 15 minutes for peroral administration of a dry extract of the leaves of Ginkgo biloba and having a total tablet weight of between 150 mg and 300 mg per 100 mg of ginkgo extract contained, prepared by a process according to any one of claims 1 to 5.

7. The rapidly disintegrating tablet of claim 6, wherein the tablet contains, in addition to the ginkgo extract, microcrystalline cellulose as a binder, a disintegration accelerator selected from croscarmellose sodium, carboxymethyl starch sodium, crospovidone or sodium starch glycolate, a flow regulating agent selected from highly dispersed silicon dioxide or precipitated silicon dioxide, and a mold release agent selected from magnesium stearate, stearic acid, behenic acid, sodium stearyl fumarate, glycerol dibehenate, calcium behenate or fumaric acid, no lactose being present, and no plant extracts or other active ingredients being present other than ginkgo extract and no excipients being present other than the excipients mentioned.

8. A rapidly disintegrating tablet having a disintegration time of at most 15 minutes for peroral administration of a dry extract from the leaves of Ginkgo biloba,
**characterized in that** the total weight of the tablet is between 150 mg and 300 mg per 100 mg of ginkgo extract contained, said tablet comprising, in addition to the ginkgo extract, microcrystalline cellulose as a binder, a disintegration accelerator selected from croscarmellose sodium, carboxymethyl starch sodium, crospovidone or sodium starch glycolate, a flow regulating agent selected from highly dispersed silicon dioxide or precipitated silicon dioxide and a mold release agent selected from magnesium stearate, stearic acid, behenic acid, sodium stearyl fumarate, glycerol dibehenate, calcium behenate or fumaric acid, wherein no lactose is contained, and no other plant extracts or other active ingredients are contained apart from the ginkgo extract and no other excipients are contained apart from the excipients mentioned.

9. The tablet according to claim 6, 7 or 8, **characterized in that** the total weight of the tablet is between 160 mg and 200 mg per 100 mg of the contained ginkgo extract.

10. The tablet according to any one of claims 6 to 9, **characterized in that** the total weight of the tablet is 175 mg per 100 mg of the contained ginkgo extract.

11. The tablet according to any one of claims 6 to 10, **characterized in that** 80 to 360 mg of dry extract from the leaves of Ginkgo biloba are contained.

12. The tablet according to any one of claims 6 to 11, **characterized in that** 220 to 260 mg of dry extract from the leaves of Ginkgo biloba are contained.

13. The tablet according to any one of claims 6 to 12, **characterized in that** 240 mg of dry extract from the leaves of Ginkgo biloba are contained.

14. The tablet according to any one of claims 6 to 13, **characterized in that** the dry extract from the leaves of Ginkgo biloba contains 22.0 to 27.0% flavonoids calculated as flavone glycosides, 2.6 to 3.2% bilobalide, 2.8 to 3.4% ginkgolides A, B and C and at most 5 ppm ginkgolic acids.

15. The tablet according to claim 6, **characterized in that** no lactose is contained.

16. The tablet according to claim 6, wherein, apart from ginkgo extract, no other plant extracts or other active ingredients are contained and, apart from the excipients mentioned, no other excipients are contained.

17. The tablet according to any one of claims 6 or 7 to 16, **characterized in that** the tablet contains microcrystalline cellulose, croscarmellose sodium, precipitated silicon dioxide and magnesium stearate.

18. The tablet according to any one of claims 6 or 7 to 17, **characterized in that** the tablet is additionally coated with a film and has a disintegration time of at most 30 minutes.

## Revendications

1. Procédé de préparation d'un comprimé à désagrégation rapide ayant un temps de désagrégation d'au plus 15 minutes pour l'administration perorale d'un extrait sec de feuilles de Ginkgo biloba et ayant un poids total de comprimé compris entre 150 mg et 300 mg pour 100 mg d'extrait de Ginkgo contenu, comprenant
(a) une première étape dans laquelle l'extrait de ginkgo est mélangé avec 6,94 mg à 27,78 mg de liant pour 100 mg d'extrait de ginkgo contenu, avec 5,56 mg à 22,22 mg d'accélérateur de décomposition pour 100 mg d'extrait de ginkgo contenu, avec 1,11 mg à 4,44 mg d'agent de régulation de l'écoulement pour 100 mg d'extrait de ginkgo contenu et avec 0,28 mg à 1,11 mg d'agent de démoulage pour 100 mg d'extrait de ginkgo contenu, puis est compactée et broyée pour obtenir un granulé concentré, et
(b) une deuxième étape de procédé, dans laquelle le granulé concentré obtenu dans l'étape (a) est mélangé avec 22,78 mg à 91,11 mg supplémentaires de liant pour 100 mg d'extrait de ginkgo contenu, avec 11,11 mg à 44,44 mg supplémentaires d'accélérateur de décomposition pour 100 mg d'extrait de ginkgo contenu, avec 0,56 mg à 2,22 mg supplémentaires d'agent de régulation de l'écoulement pour 100 mg d'extrait de ginkgo contenu et avec 1,67 mg à 6,67 mg supplémentaires d'agent de démoulage pour 100 mg d'extrait de ginkgo contenu et est pressé en comprimés,
dans lequel la quantité totale d'adjuvants dans l'étape (a) est de 13,89 mg à 55,56 mg pour 100 mg d'extrait de ginkgo contenu et dans l'étape (b) de 36,11 mg à 144,44 mg pour 100 mg d'extrait de ginkgo contenu et
dans lequel le liant dans l'étape (a) et dans l'étape (b) est de la cellulose microcristalline, l'accélérateur de décomposition dans l'étape (a) et dans l'étape (b) est choisi indépendamment parmi la croscarmellose sodique, le carboxyméthylamidon sodique, la crospovidone ou l'amidon glycolate sodique, l'agent de régulation de l'écoulement dans l'étape (a) et dans l'étape (b) est choisi indépendamment parmi la silice hautement dispersée ou la silice précipitée, et l'agent de démoulage dans l'étape (a) et dans l'étape (b) est choisi indépendamment parmi le stéarate de magnésium, l'acide stéarique, l'acide béhénique, le fumarate de stéaryle sodique, le dibéhénate de glycérol, le béhénate de calcium ou l'acide fumarique.

2. Procédé selon la revendication 1 pour la préparation d'un comprimé à désagrégation rapide ayant un temps de désagrégation d'au plus 15 minutes pour l'administration perorale d'un extrait sec de feuilles de Ginkgo biloba et ayant un poids total de comprimé compris entre 160 mg et 200 mg pour 100 mg d'extrait de Ginkgo contenu, **caractérisé en ce que**
(a) l'extrait de ginkgo est mélangé, dans une première étape du procédé, avec 8,33 mg à 13,89 mg de liant pour 100 mg d'extrait de ginkgo contenu, avec 6,67 mg à 11,11 mg d'accélérateur de décomposition pour 100 mg d'extrait de ginkgo contenu, avec 1,33 mg à 2,22 mg d'agent de régulation de l'écoulement pour 100 mg d'extrait de ginkgo contenu et 0,33 mg à 0,56 mg d'agent de démoulage pour 100 mg d'extrait de ginkgo contenu, puis est compactée et broyée pour obtenir un granulé concentré, et
(b) le granulé concentré obtenu dans l'étape (a) dans une deuxième étape du procédé est mélangé avec 27,33 mg à 45,56 mg supplémentaires de liant pour 100 mg d'extrait de ginkgo contenu, avec 13,33 mg à 22,22 mg supplémentaires d'accélérateur de décomposition pour 100 mg d'extrait de ginkgo contenu, avec 0,67 mg à 1,11 mg supplémentaire d'agent de régulation de l'écoulement pour 100 mg d'extrait de ginkgo contenu et avec 2,00 mg à 3,33 mg supplémentaires d'agent de démoulage pour 100 mg d'extrait de ginkgo contenu et est pressé en comprimés,
dans lequel la quantité totale d'adjuvants dans l'étape (a) est de 16,67 mg à 27,78 mg pour 100 mg d'extrait de ginkgo contenu et dans l'étape (b) de 43,33 mg à 72,22 mg d'extrait de ginkgo contenu et
dans lequel le liant dans l'étape (a) et dans l'étape (b) est de la cellulose microcristalline, l'accélérateur de décomposition dans l'étape (a) et dans l'étape (b) est choisi indépendamment parmi la croscarmellose sodique, le carboxyméthylamidon sodique, la crospovidone ou l'amidon glycolate sodique, l'agent de régulation de l'écoulement dans l'étape (a) et dans l'étape (b) est choisi indépendamment parmi la silice hautement dispersée ou la silice précipitée, et l'agent de démoulage dans l'étape (a) et dans l'étape (b) est choisi indépendamment parmi le stéarate de magnésium, l'acide stéarique, l'acide béhénique, le fumarate de stéaryle sodique, le dibéhénate de glycérol, le béhénate de calcium ou l'acide fumarique.

3. Procédé selon l'une des revendications 1 ou 2, dans lequel l'accélérateur de décomposition dans l'étape (a) et dans l'étape (b) est la croscarmellose sodique, l'agent de régulation de l'écoulement dans l'étape (a) et dans l'étape (b) est la silice précipitée et l'agent de démoulage dans l'étape (a) et dans l'étape (b) est le stéarate de magnésium.

4. Procédé selon l'une quelconque des revendications 1 à 3, dans lequel le compactage à l'étape (a) est effectué par compactage à rouleaux.

5. Procédé selon l'une quelconque des revendications 1 à 4, dans lequel, en dehors de l'extrait de ginkgo, on n'utilise pas d'autres extraits de plantes ou d'autres substances actives et, en dehors des adjuvants mentionnés, on n'utilise pas d'autres adjuvants.

6. Comprimé à désintégration rapide ayant un temps de désintégration d'au plus 15 minutes pour l'administration perorale d'un extrait sec de feuilles de Ginkgo biloba et ayant un poids total de comprimé compris entre 150 mg et 300 mg pour 100 mg d'extrait de Ginkgo contenu, préparé selon un procédé selon l'une quelconque des revendications 1 à 5.

7. Comprimé à désintégration rapide selon la revendication 6, dans lequel le comprimé contient, outre l'extrait de ginkgo, de la cellulose microcristalline en tant que liant, un accélérateur de désintégration choisi parmi la croscarmellose sodique, le carboxyméthylamidon sodique, la crospovidone ou l'amidon glycolate sodique, un agent de régulation de l'écoulement choisi parmi la silice hautement dispersée ou la silice précipitée et un agent de démoulage choisi parmi le stéarate de magnésium, de l'acide stéarique, de l'acide béhénique, du stéarylfumarate de sodium, du dibéhénate de glycérol, du béhénate de calcium ou de l'acide fumarique, aucun lactose n'étant contenu et, à l'exception de l'extrait de ginkgo, aucun autre extrait végétal ou autre substance active n'étant contenu et aucun autre adjuvant n'étant contenu à l'exception des adjuvants mentionnés.

8. Comprimé à désintégration rapide ayant un temps de désintégration d'au plus 15 minutes pour l'administration perorale d'un extrait sec de feuilles de Ginkgo biloba, **caractérisé en ce que** le poids total du comprimé est compris entre 150 mg et 300 mg pour 100 mg d'extrait de Ginkgo contenu, le comprimé contenant, outre l'extrait de Ginkgo, de la cellulose microcristalline comme liant, un accélérateur de désintégration choisi parmi la croscarmellose sodique, le carboxyméthylamidon sodique, crospovidone ou glycolate d'amidon sodique, un agent de régulation de l'écoulement choisi parmi le dioxyde de silicium hautement dispersé ou le dioxyde de silicium précipité et un agent de démoulage choisi parmi le stéarate de magnésium, l'acide stéarique, l'acide béhénique, le fumarate de stéaryle sodique, le dibéhénate de glycérol, le béhénate de calcium ou l'acide fumarique, aucun lactose n'étant contenu, et aucun autre extrait végétal ou autre substance active n'étant contenu en dehors de l'extrait de ginkgo et aucun autre adjuvant n'étant contenu en dehors des adjuvants mentionnés.

9. Comprimé selon la revendication 6, 7 ou 8, **caractérisé en ce que** le poids total du comprimé est compris entre 160 mg et 200 mg pour 100 mg d'extrait de ginkgo contenu.

10. Comprimé selon l'une des revendications 6 à 9, **caractérisé en ce que** le poids total du comprimé est de 175 mg pour 100 mg d'extrait de ginkgo contenu.

11. Comprimé selon l'une des revendications 6 à 10, **caractérisé en ce qu'**il contient de 80 à 360 mg d'extrait sec de feuilles de Ginkgo biloba.

12. Comprimé selon l'une des revendications 6 à 11, **caractérisé en ce qu'**il contient 220 à 260 mg d'extrait sec de feuilles de ginkgo biloba.

13. Comprimé selon l'une des revendications 6 à 12, **caractérisé en ce qu'**il contient 240 mg d'extrait sec de feuilles de ginkgo biloba.

14. Comprimé selon l'une des revendications 6 à 13, **caractérisé en ce que** l'extrait sec de feuilles de Ginkgo biloba contient 22,0 à 27,0 % de flavonoïdes calculés sous forme de glycosides de flavone, 2,6 à 3,2 % de bilobalide, 2,8 à 3,4 % de ginkgolides A, B et C et au plus 5 ppm d'acides ginkgoliques.

15. Comprimé selon la revendication 6, **caractérisé en ce qu'**il ne contient pas de lactose.

16. Comprimé selon la revendication 6, dans lequel, à l'exception de l'extrait de ginkgo, aucun autre extrait de plante ou autre substance active n'est contenu et, à l'exception des adjuvants mentionnés, aucun autre adjuvant n'est contenu.

17. Comprimé selon l'une des revendications 6 ou 7 à 16, **caractérisé en ce que** le comprimé contient de la cellulose microcristalline, de la croscarmellose sodique, du dioxyde de silicium précipité et du stéarate de magnésium.

18. Comprimé selon l'une des revendications 6 ou 7 à 17, **caractérisé en ce que** le comprimé est en outre enrobé d'un film et présente un temps de désagrégation d'au plus 30 minutes.
